# EUROPEAN PATENT SPECIFICATION

(11) **EP 4 161 511 B1**
(45) Date of publication and mention of the grant of the patent: **29.04.2026**
(21) Application number: 21817859.8
(22) Date of filing: 26.05.2021
(51) Int. Cl.: A61K 31/44, A61K 31/4439, C07D 401/12, A61P 35/00

(54) **NOVEL SMALL MOLECULES FOR TARGETED DEGRADATION OF UNTARGETABLE KRAS IN CANCER THERAPY**
NEUARTIGE KLEINE MOLEKÜLE ZUM GEZIELTEN ABBAU VON NICHT ABZIELBAREN KRAS IN DER KREBSTHERAPIE
NOUVELLES PETITES MOLÉCULES POUR LA DÉGRADATION CIBLÉE DE KRAS NE POUVANT ÊTRE CIBLÉ DANS UNE THÉRAPIE ANTICANCÉREUSE

(30) Priority: 04.06.2020 IN 202041023434
(43) Date of publication of application: 12.04.2023
(73) Proprietor: Pillai Universal LLC, Carson City, NV 92590 (US)
(72) Inventor: PILLAI, Baskaran, Pittsburgh, Pennsylvania 15238 (US); M.G., Dinesh, Chennai, 600053 (IN)
(74) Representative: Cooley (UK) LLP
(86) International application number: PCT/IB2021/054591
(87) International publication number: WO 2021/245499

(56) References cited:
- WO-A1-01/90101
- WO-A1-03/037891
- WO-A1-2011/130628
- WO-A1-2011/130628
- WO-A2-2007/075896
- DATABASE PUBCHEM COMPOUND 23 June 2018 (2018-06-23), ANONYMOUS: "SCHEMBL20094434", XP055879859, retrieved from PUBCHEM Database accession no. 374716721
- DATABASE PUBCHEM COMPOUND 17 March 2015 (2015-03-17), ANONYMOUS: "SID:245134309", XP055879869, retrieved from PUBCHEM Database accession no. 245134309

## Description

### FIELD OF THE INVENTION:

The present invention relates to pharmaceutical molecules. More particularly the present invention relates to a novel anti-cancer activity exhibiting compounds for treating subjects with chronic disorder.

### BACKGROUND OF THE INVENTION:

Cancer is a major health concern and a leading cause of death. Cancer prognosis is intimately linked to disease stage. At the most advanced stages, when metastases occur, prognosis is generally aggravated, and treatments are most likely to fail. Genomic instability and mutations were pointed out as the main mechanisms underlying the acquisition of these hallmark features. One of the most frequently mutated oncogenes in cancer is KRAS. KRAS gene encodes a small GTPase, which cycles between GDP and GTP-bound states as a consequence of the stimulation of certain cell surface receptors, such as the EGFR. The most common KRAS mutations include G12C, G12D, G12R, G12S, G12 V, G13D and Q61H [Meng et al., 2013]. The most common mutation sites are in codon 12 (80% of tumors), codon 13, and codon 61 [Friday et al., 2015]. Activating KRAS mutations are most prevalent in pancreatic carcinomas (72% - 90%), colorectal carcinomas (28% - 57%), and lung carcinomas (15% - 50%). In lung cancers, KRAS mutations are detected in 15% to 20% of non small cell lung carcinomas (NSCLCs), are most frequent in adenocarcinomas (30% - 50%), are rare in small cell lung cancers, and are more frequent in smokers.

KRAS (v-Ki-ras2 Kirsten rat sarcoma viral oncogene homolog) is an oncogenic driver with mutations in 30% of non-small cell lung cancer (NSCLC). However, there is no effective clinical drug even though it has been identified as an oncogene for 30 years. KRAS is one of the most frequently mutated oncogenes in cancer, being a potent initiator of tumorigenesis, a strong inductor of malignancy, and a predictive biomarker of response to therapy. In addition to holding this distinction, unsuccessful attempts to target this protein have led to the characterization of RAS as 'undruggable'. RAS signaling networks are built at the membrane of cells, bridging extracellular cues into cellular events, such as cell growth, proliferation, differentiation, and survival [Cox et al., 2015], having a decisive role in the transition of healthy cells to cancer [Welsch ME et al., 2017]. All these findings have led the scientific community to exploit RAS or its downstream effectors as therapeutic targets, hoping to impair tumor growth and survival [Affolter et al., 2012]. However, no effective KRAS directed signaling inhibition has been until now successfully achieved until now. Mutations that render KRAS constitutively active will lead to uncontrolled cell growth and cancer. However, despite aggressive efforts in recent years, there are no drugs on the market that directly target KRAS and inhibit its aberrant functions [Westcott et al., 2015].

However, pre-clinical studies indicate that "K-Ras addiction" is reduced in mesenchymal cancer cells implicating that direct KRAS inhibition may not be efficacious in all patients [Yin et al., 2019]. Alternatively, inhibitors targeting kinases downstream of KRAS, such as BRAF and MEK, have shown promising activity in metastatic melanoma but were largely ineffective in KRAS mutation in cancer in combination with chemotherapy [Janne et al., 2017]. Apart from intrinsic resistance e.g. due to mesenchymal cancer cell differentiation [Peng, et al., 2019], efficacy of MEK inhibitors is limited by the development of acquired resistance [Haigis et al., 2017]. Another factor contributing to the difficulty to treat cancer cells is the heterogeneity of different KRAS mutations which are defined by the respective amino acid substitutions. These change the protein structure and GTPase activity of KRAS and substantially affect the tumour biology and response to chemotherapy [Ambrogio et al., 2018]. WO 2011/130628 discloses compounds for the treatment of cancers having *K-ras* mutations. Hence, an unmet need remains to develop more efficacious targeted treatment strategies for patients with KRAS mutant lung cancer.

### References

1. Overmeyer JH, Maltese WA. Death pathways triggered by activated Ras in cancer cells. Front Biosci (Landmark Ed). 2011;16:1693-713.
2. Welsch ME, Kaplan A, Chambers JM, Stokes ME, Bos PH, Zask A, Zhang Y, Sanchez-Martin M, Badgley MA, Huang CS, et al. Multivalent small-molecule pan-RAS inhibitors. Cell. 2017;168:878-889 e829
3. Affolter A, Drigotas M, Fruth K, Schmidtmann I, Brochhausen C, Mann WJ, Brieger J. Increased radioresistance via G12S K-Ras by compensatory upregulation of MAPK and PI3K pathways in epithelial cancer. Head Neck. 2012.
4. Meng D, Yuan M, Li X, Chen L, Yang J, Zhao X, Ma W, Xin J. Prognostic value of K-RAS mutations in patients with non-small cell lung cancer: a systematic review with meta-analysis. Lung Cancer. 2013;81:1-10.
5. A.D. Cox, C.J. Der, M.R. Philips Targeting RAS membrane association: back to the future for anti-RAS drug discovery? Clin Cancer Res, 21 (2015), pp. 1819-1827
6. P.M. Westcott, K.D. Halliwill, M.D. To, M. Rashid, A.G. Rust, T.M. Keane, et al. The mutational landscapes of genetic and chemical models of KRAS-driven lung cancer Nature, 517 (2015), pp. 489-492
7. Friday BB,Adjei AA. KRAS as a target for cancer therapy. BiochimBiophysi Acta. 2005; 1756: 127-144.
8. P.A. Janne, M.M. van den Heuvel, F. Barlesi, M. Cobo, J. Mazieres, L. Crino, et al. Selumetinib plus docetaxel compared with docetaxel alone and progression-free survival in patients with KRAS-Mutant advanced non-small cell lung cancer: the SELECT-1 randomized clinical trial JAMA, 317 (18) (2017), pp. 1844-1853
9. D.H. Peng, S.T. Kundu, J.J. Fradette, L. Diao, P. Tong, L.A. Byers, et al. ZEB1 suppression sensitizes kras mutant cancers to mek inhibition by an IL17RD-dependent mechanism Sci Transl Med, 11 (483) (2019)
10. C. Ambrogio, J. Kohler, Z.W. Zhou, H. Wang, R. Paranal, J. Li, et al. KRAS dimerization impacts mek inhibitor sensitivity and oncogenic activity of mutant kras Cell, 172 (4) (2018) 857-68 e15
11. K.M. Haigis KRAS alleles: the devil is in the detail Trends Cancer, 3 (10) (2017), pp. 686-697
12. N. Yin, Y. Liu, A. Khoor, X. Wang, E.A. Thompson, M. Leitges, et al. Protein kinase ciota and wnt/beta-catenin signaling: alternative pathways to kras/trp53-driven lung adenocarcinoma Cancer Cell, 36 (2) (2019)156-67.e7

### OBJECT OF THE INVENTION:

The main object of the present invention is to develop novel anti-cancer activity exhibiting compounds for treating subjects with chronic disorder.

Another object of the present invention is to synthesize the novel anti-cancer activity exhibiting compounds for treating subjects with chronic disorder.

Yet another object of the present invention is to synthesize anti-cancer activity exhibiting compounds which selectively target Cancer stem cells and efficient in KRAS protein degradation.

Further object of the present invention is to utilize the synthesized novel anti-cancer activity exhibiting compounds for treating subjects with chronic disorder.

A further object of the present invention is to provide pharmaceutical compositions comprising an effective amount of one or more of the compounds described herein.

Yet another object of the present invention is to provide methods of modulating, inhibiting, or degrading KRAS in a cell, or any combination thereof, by administering to the cell an effective amount of one or more of the compounds or pharmaceutical compositions described herein.

Yet another object provided herein is a method of treating a disease, disorder, or condition mediated by KRAS in a subject in need thereof, comprising administering to the subject an effective amount of one or more of the compounds or pharmaceutical compositions described herein.

In yet another object provided herein is an article of manufacture (for example, a kit) comprising (i) an effective amount of one or more of the compounds or pharmaceutical compositions described herein, and (ii) instructions for use in treating a disease, disorder, or condition mediated by KRAS.

### BRIEF DESCRIPTION OF DRAWINGS:

The foregoing and following information as well as other features of this disclosure will become more fully apparent from the following description and appended claims, taken in conjunction with the accompanying drawings. Understanding that these drawings depict only several embodiments in accordance with the disclosure and are, therefore, not to be considered limiting of its scope, the disclosure will be described with additional specificity and detail through use of the accompanying drawings.
Figure 1 depicts the spectrum of compound I
   a. Mass Spectrum
   b. IR Spectrum
   c. ¹H NMR Spectrum
   d. ¹³C NMR Spectrum
Figure 2 depicts the spectrum of compound II
   a. Mass Spectrum
   b. IR Spectrum
   c. ¹H NMR Spectrum
   d. ¹³C NMR Spectrum
Figure 3 depicts the spectrum of compound III
   a. Mass Spectrum
   b. IR Spectrum
   c. ¹H NMR Spectrum
   d. ¹³C NMR Spectrum
Figure 4 depicts the spectrum of compound IV
   a. Mass Spectrum
   b. IR Spectrum
   c. ¹H NMR Spectrum
   d. ¹³C NMR Spectrum
Figure 5 depicts the spectrum of compound V
   a. Mass Spectrum
   b. IR Spectrum
   c. ¹H NMR Spectrum
   d. ¹³C NMR Spectrum
Figure 6 depicts the compound I induces apoptosis in KRAS-dependent cancer cell line Figure-6 (A) - Morphological changes after 24 h exposure to compound I (0, 50, 100 and 250nm) were captured by an Epifluroscent microscope with ×100 magnification, scale bar: 20 µm.
   Figure-6 (B) - Cells were treated with 250nm of Compound I for 24 h and apoptosis levels were quantitatively measured with flow cytometry after staining cells with Annexin V/ propidium iodide (PI), and triplicate data were plotted as bar chart diagram.
   Figure-6 (C) - Representative western blot data of different apoptosis-related protein (PARP, Bcl-2, and Bax) confirmed induction of cellular apoptosis after treatment (0, 50, 100 and 250nm) for 24 h. Densitometry of the ratio of Bax/Bcl2 and cleaved PARP were shown as bar chart.
   All data were representative of at least three independent experiments and presented as mean ± SD, *P < 0.05, **P < 0.01, ***P < 0.001 compared with control.

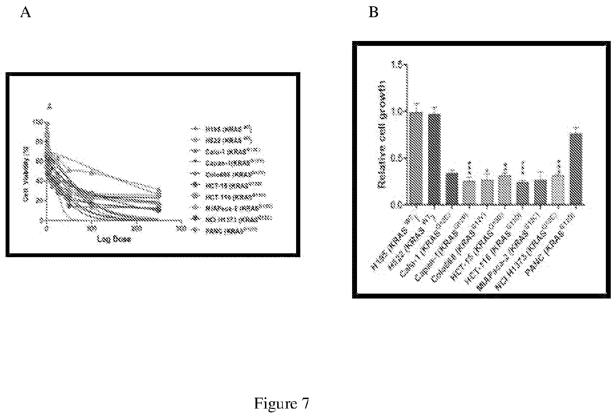

Figure 7 depicts the compound I inhibit KRAS mutant expressing cancer cells
   Figure 7A - Proliferation profile of KRAS-expressing cancer clls upon treatment by increasing concentration of compound compound I and monitored by Cell viability assay.
   Figure 7B - Relative growth of the KRAS mutant and wild type cancer cells after treatment with IC 50 concentration of compound compound I.
   Data are shown as mean ± SD; significance waqs estimated by one-way ANOVA with respect to the sata for SKLU-1: * = P < 0.02, **=P < 0.005, ***=P < 0.0001.

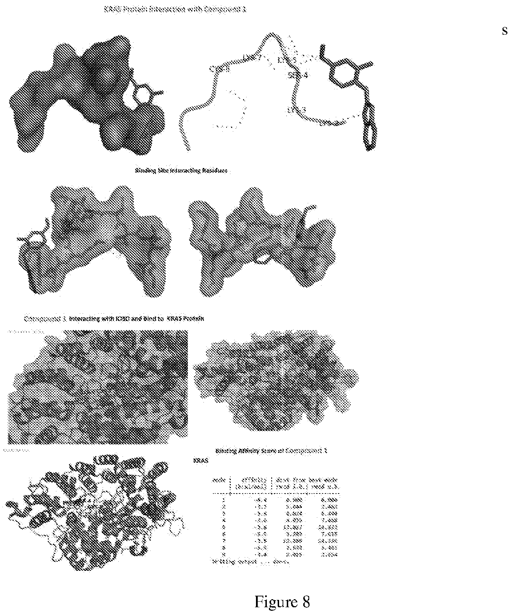

Figure 8 depicts theStructural modelling of compound I

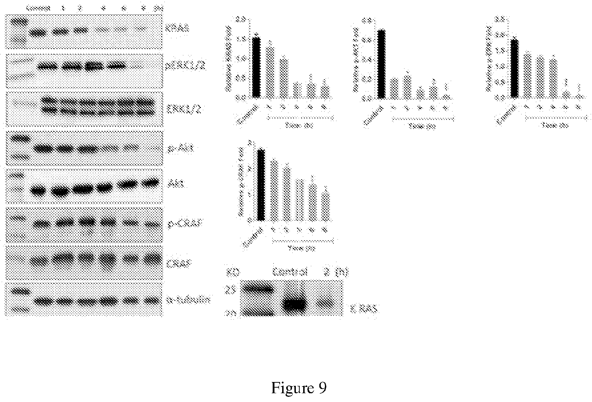

Figure 9 depicts the Compound compound I Blocked GTP-KRAS Formation in KRAS Mutant cells

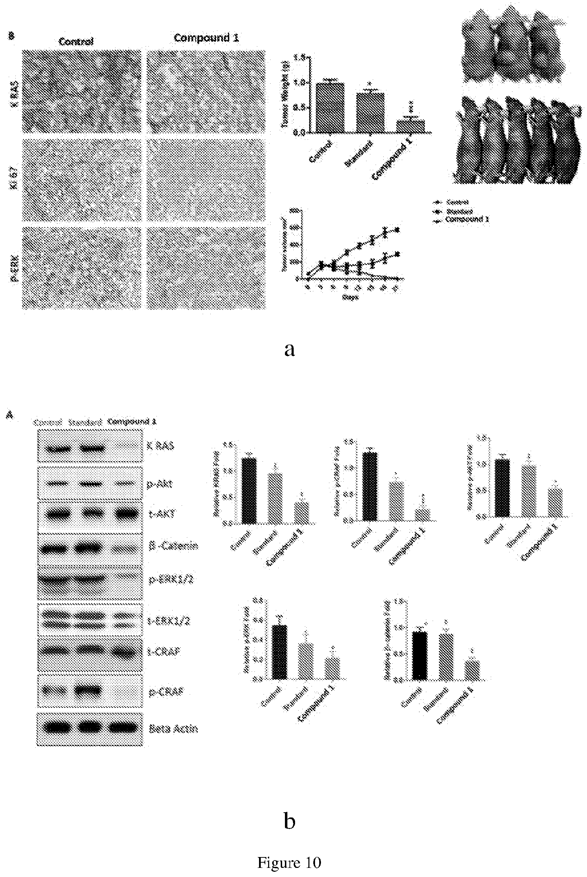

Figure 10 depicts the compound I suppresses tumor growth in xenograft Model and also depicts the Western blotting analysis of KRAS mutant proteins in In vivo animal model

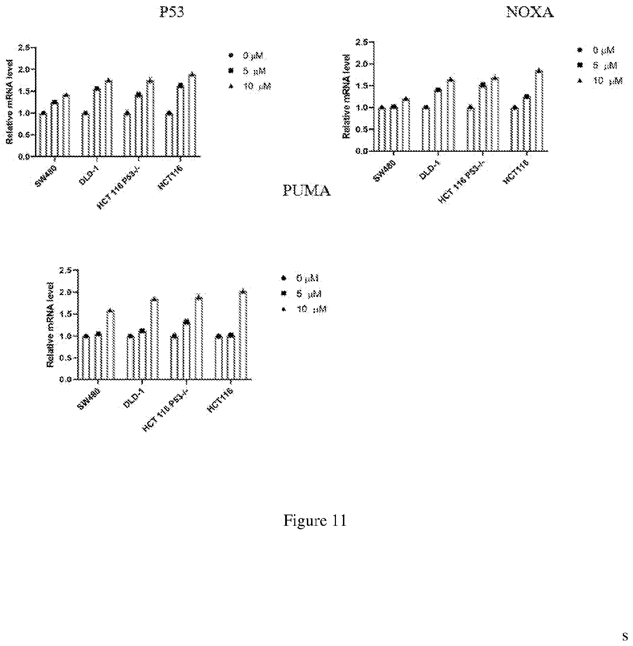

Figure 11 depicts compound I Targeting p53 (mRNA levels of p53, Puma, and Noxa in cells at 6 hours after compound I treatment. mRNA levels were quantified by qRT-PCR. Data were normalized to GAPDH expression and plotted relative to cells treated with DMSO as control)

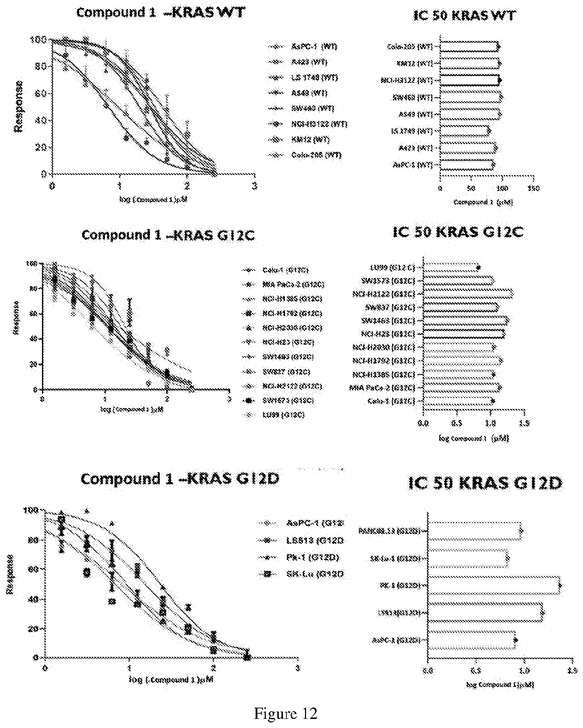

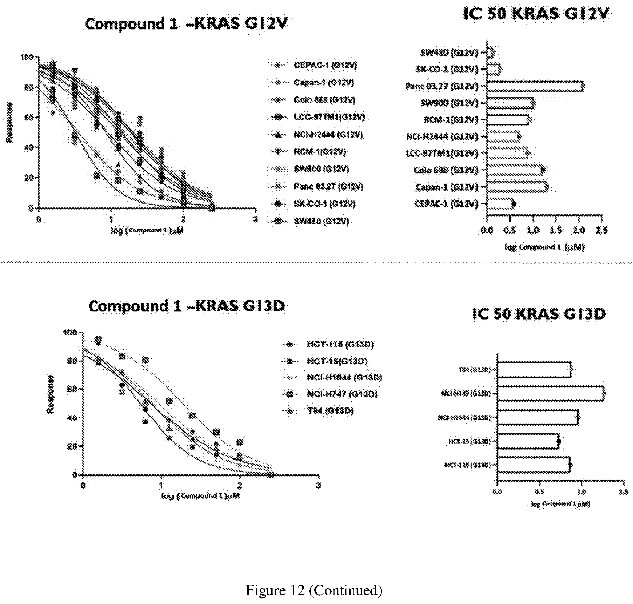

Figure 12 depicts that Compound 1 was found to significantly inhibit cancer cell viability in all tested mutant cells in a dose-dependent manner.

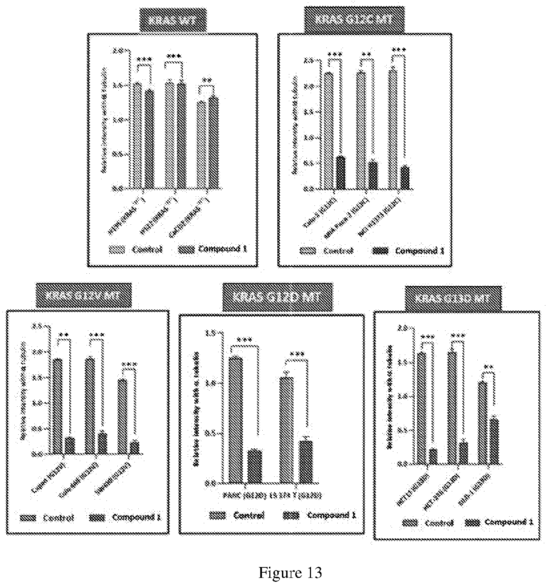

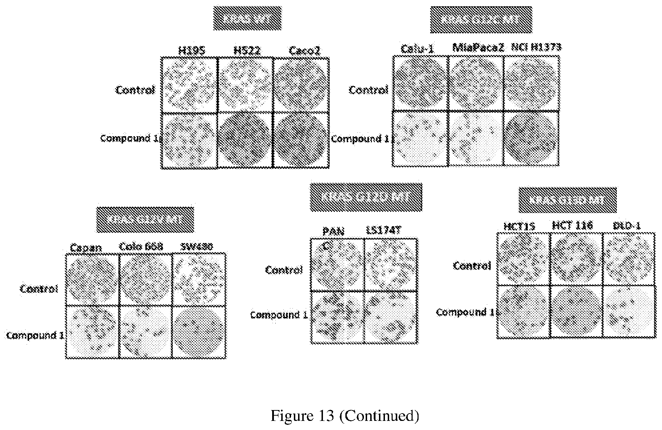

Figure 13 depicts that Compound 1 was found to selectively inhibit the formation of GTP-KRAS (relative to the total amount of KRAS) in KRAS G12C, G12V, G12D, and G13D mutant cells.

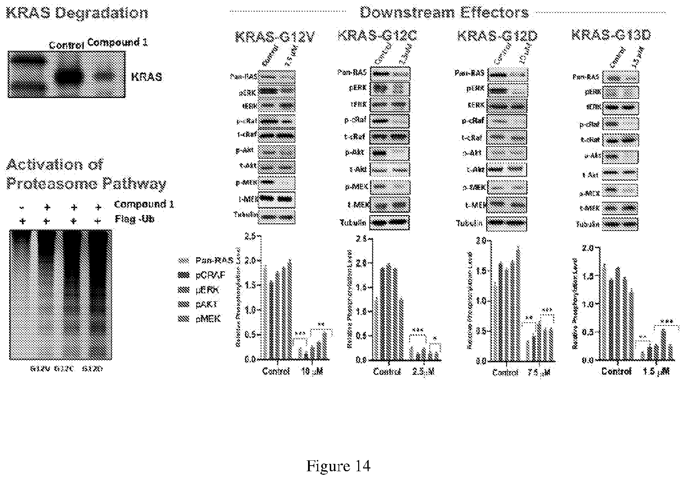

Figure 14 depicts that Compound 1blocked Downstream effector of RAS signaling in KRAS Mutant cells.

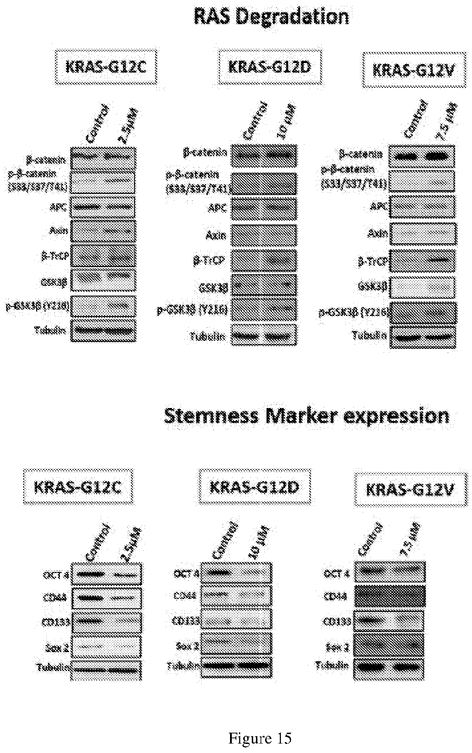

Figure 15 depicts that Compound 1 was found to degrade RAS and inhibits Stemness Marker expression.

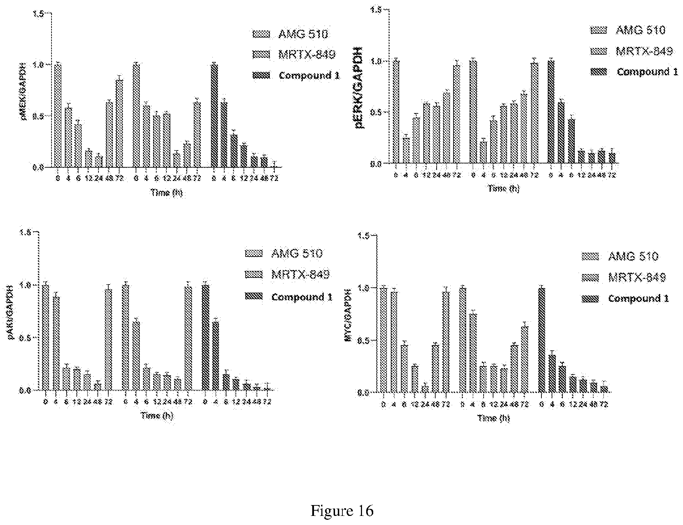

Figure 16 depicts a comparison of RAS reactivation in KRAS G12C mutant cell lines following treatment with Compound 1 and two known KRAS G12C inhibitors.

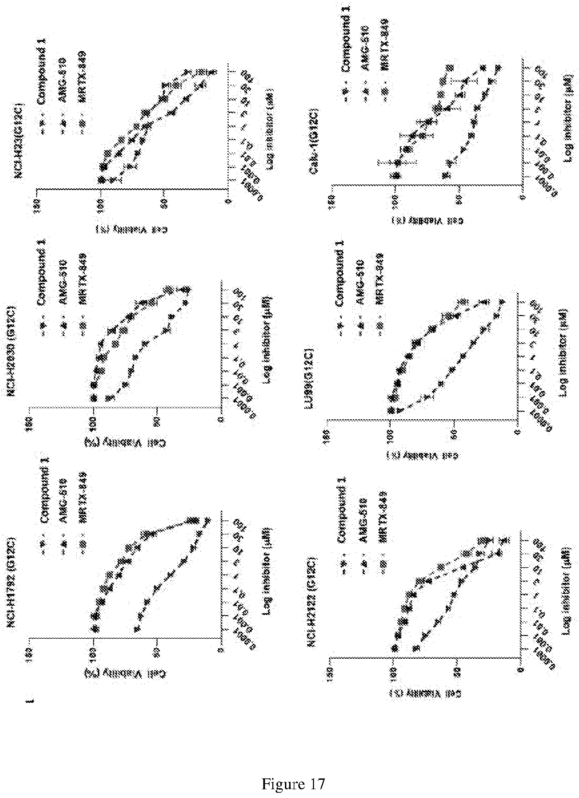

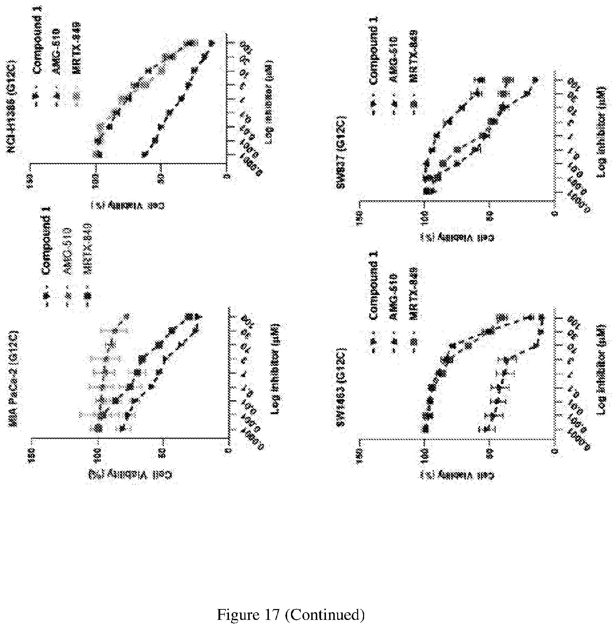

Figure 17 depicts a comparison of cell viability in KRAS G12C mutant cell lines following treatment with Compound 1 and two known KRAS G12C inhibitors.

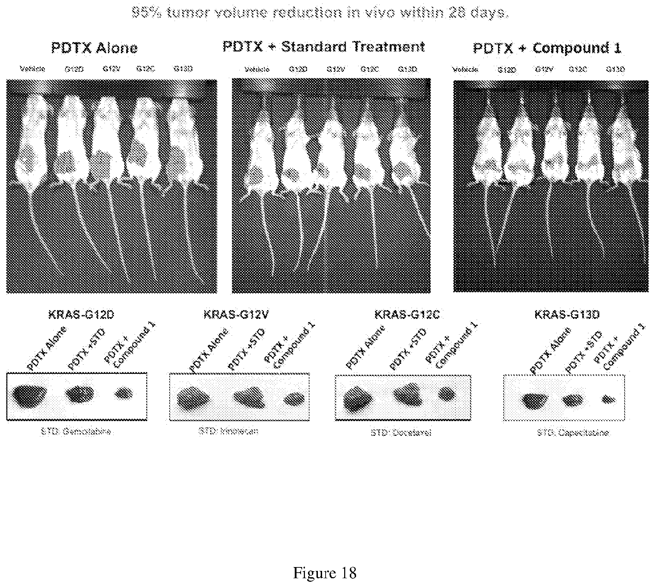

Figure 18 depicts regression of all mutant-KRAS-driven cancer in PDTX Animal Model. Treatment with Compound 1 showed a 95% reduction in tumor growth within 28 days and no evidence of relapse for 6-8 months.

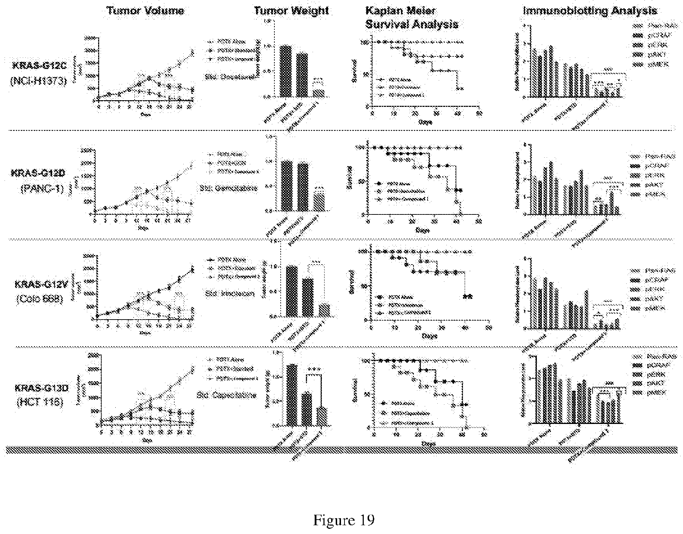

Figure 19 depicts regression of all mutant-KRAS-driven cancer in PDTX Animal Model. Treatment with Compound 1 showed a 95% reduction in tumor growth within 28 days and no evidence of relapse for 6-8 months.

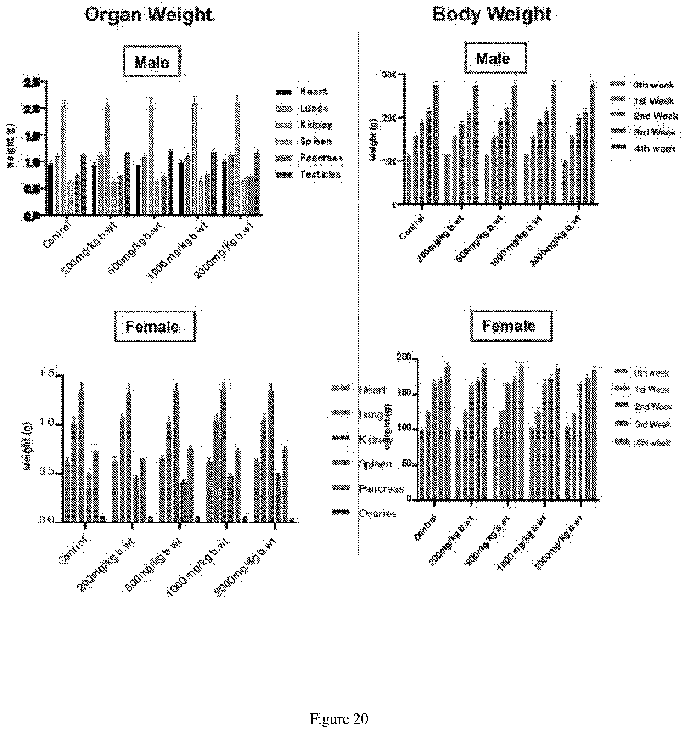

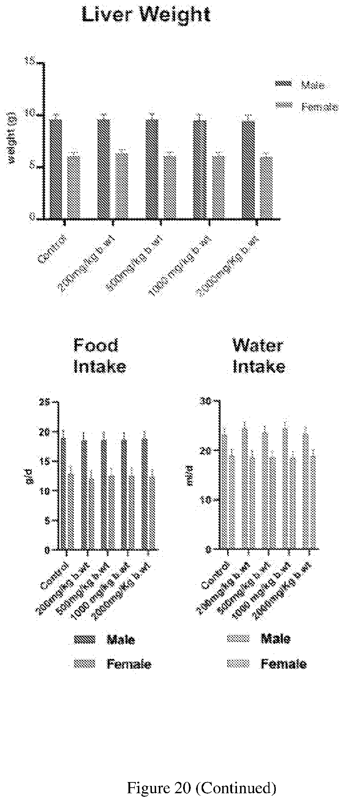

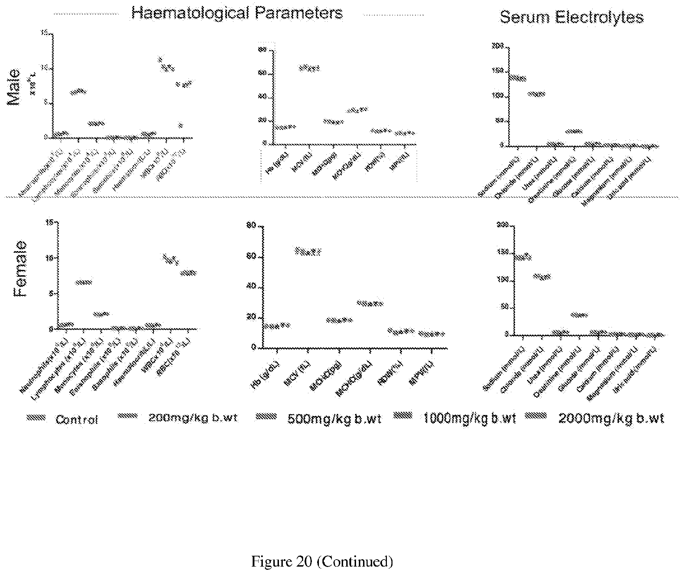

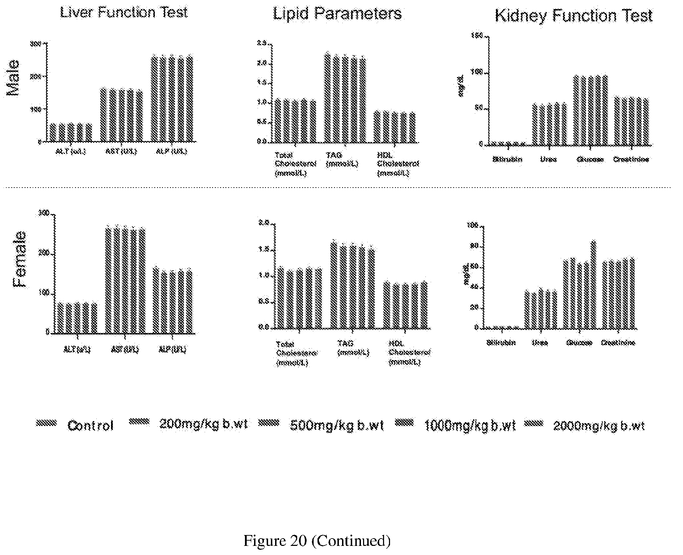

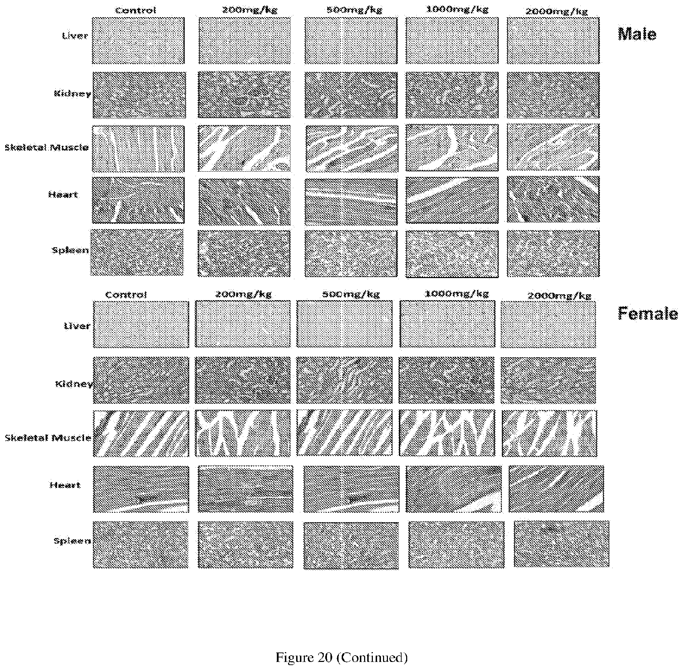

Figure 20 depicts the results of *in vivo* toxicity studies of Compound 1. Organ weight, Body weight, liver weight, haematological parameters, serum electrolyte LFT, KFT, Lipid parameters found to be normal range compared with the controlled group. Histopathology of harvested normal tissues (heart, liver, spleen, skeletal muscle, kidney, and intestine) revealed no evidence of normal tissue toxicities after treatment with specific doses of Compound 1.

### SUMMARY OF THE INVENTION:

The present invention provides a compound of formula 1 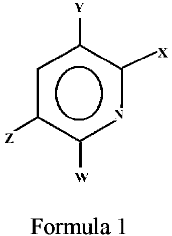
or a pharmaceutically acceptable salt thereof, or combination thereof, in which
X is C₁-C₅ alkyl;
Y is carboxyl group bonded to C₁-C₅ alkyl;
Z is carbonyl group bonded to 5-carbamoyl-2,3-dihydro-1H-pyrrole; and
W is H.

In some embodiments, the compound is

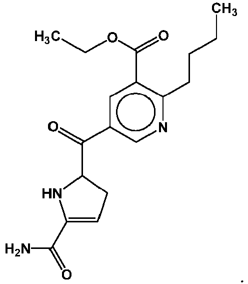

The present invention also provides a pharmaceutical composition comprising an effective amount of a compound of the invention and a pharmaceutically acceptable carrier.

The present invention also provides a compound or pharmaceutical composition of the present invention, for use in a method of treating a disease, disorder, or condition mediated by KRAS, wherein the disease, disorder, or condition is cancer. In some embodiments, the cancer is non-small-cell lung cancer, triple-negative breast cancer, colorectal cancer, pancreatic cancer, or a combination thereof.

### DETAILED DESCRIPTION OF THE INVENTION:

### Definitions:

"Alkyl" or "alkyl group" refers to a fully saturated, straight or branched hydrocarbon chain having from one to fifteen carbon atoms, and which is attached to the rest of the molecule by a single bond. Unless stated otherwise specifically in the specification, an alkyl group can be optionally substituted.

"Carbonyl group" refers to

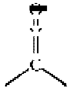

Carboxyl group refers to 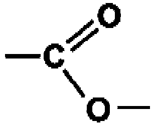 As used herein, the symbol 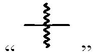 (hereinafter can be referred to as "a point of attachment bond") denotes a bond that is a point of attachment between two chemical entities, one of which is depicted as being attached to the point of attachment bond and the other of which is not depicted as being attached to the point of attachment bond.

Compounds provided herein that have the same molecular formula but which differ in the type or sequence of bonding of their atoms or the arrangement of their atoms in space are referred to as "isomers." Isomers that differ in the arrangement of their atoms in space are termed "stereoisomers."The compounds of the present disclosure may also exist in different tautomeric forms. The term "tautomer" refers to structural isomers of which are interconvertible. For example, in one variation, a compound provided herein may exhibit keto-enol tautomerism. In another variation, a compound provided herein may exhibit imine-enamine tautomerism.

"Pharmaceutically acceptable salts" include, when appropriate, pharmaceutically acceptable base addition salts and acid addition salts, As used herein, the term "pharmaceutically acceptable" infers that the salt is not biologically or otherwise undesirable; for example, the material may be added to a pharmaceutical composition and administered to a subject without causing significant undesirable effects.

The term "therapeutically effective" applied to dose or amount refers to that quantity of a compound or pharmaceutical formulation that is sufficient to result in a desired clinical benefit after administration to a patient in need thereof. It is to be understood that an effective amount may be in one or more doses, e.g., a single dose or multiple doses may be needed to achieve the desired treatment endpoint.

The terms "treating" or "treatment", as used herein, refer to a method or procedure for obtaining beneficial or desired results-for example, clinical results. Beneficial or desired results may include: (1) alleviating one or more symptoms caused by or associated with a disease, disorder, or condition; (2) reducing the extent of the disease, disorder, or condition; (3) slowing or stopping the development or progression of one or more symptoms caused by or associated with the disease, disorder, or condition (for example, stabilizing the disease, disorder, or condition); and (4) relieving the disease, for example, by causing the regression of one or more clinical symptoms (e.g., ameliorating the disease state, enhancing the effect of another medication, delaying or stopping the progression of the disease, increasing the quality of life, and/or prolonging survival rates).

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the scope of the subject matter presented herein. It will be readily understood that the aspects of the present disclosure, as generally described herein, and illustrated in the figures, can be arranged, substituted, combined, separated, and designed in a wide variety of different configurations, all of which are explicitly contemplated herein.

The present invention discloses novel anti-cancer activity exhibiting compounds for use in treating subjects with chronic disorder. In particular, the present invention provides a compound of formula 1: 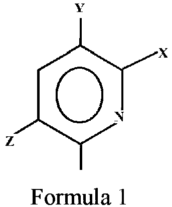
or a pharmaceutically acceptable salt thereof, or combination thereof, in which
X is C₁-C₅ alkyl;
Y is carboxyl group bonded to C₁-C₅ alkyl;
Z is carbonyl group bonded to 5-carbamoyl-2,3-dihydro-1H-pyrrole; and
W is H.

In some embodiments, the compound is

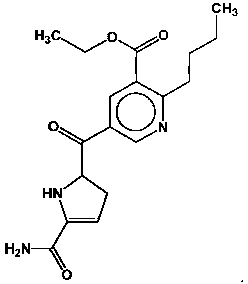

Also disclosed herein are compounds of the following structure: 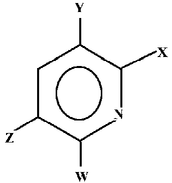
or any derivative thereof, or a stereoisomer or tautomer thereof, pharmaceutically acceptable salt thereof, or combination thereof
in which
X is H or alkyl group;
Y is carbonyl group bonded to substituted/unsubstituted homo/hetero cyclic ring with atleast one double bond or carboxyl group bonded to alkyl group;
Z is carbonyl/carboxyl group bonded to substituted/unsubstituted homo/hetero cyclic ring with atleast one double bond or alkene or o-alkyl substituted carbamate or carbonyl group bonded to branched alkyl group and
W is H or amide group or carbonyl group bonded to branched alkyl group.

In some such compounds, X is selected from a group comprising of H or C₁-C₅ alkyl group
In some such compounds, Y is selected from a group comprising of
i. Carbonyl group bonded to cyclo pent-3-ene;
ii. Carboxyl group bonded to C₁-C₅ alkyl group;
iii. Carbonyl group bonded to 5 carbamoyl, 4,5 dihydro 1H pyrrole
iv. Carbonyl group bonded to 4,5 dihydro 1H pyrrole 2 carbamoyl

In some such compounds, Z is selected from a group comprising of
i. o- C₁-C₅ alkyl carbamate;
ii. carbonyl group bonded to 5 carbamoyl, 2,3, dihydro 1H pyrrole;
iii. carboxyl group bonded to pyridine,
iv. C₁-C₅ alkene;
v. Carbonyl group bonded to C₁-C₆ branched alkyl group;

In some such compounds, W is selected from a group comprising of Amide group or H or Carbonyl group bonded to C₁-C₅ branched alkyl group.

In some such compounds,
XisH
Y is Carbonyl group bonded to cyclo pent-3-ene
Z is o-methyl carbamate
W is amide group

In some such compounds,
XisH
Y is 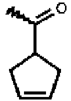
Z is 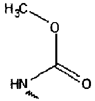
W is

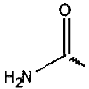

In other such compounds,
X is butyl group
Y is Carboxyl group bonded to ethyl group
Z is carbonyl group bonded to 5 carbamoyl, 2,3, dihydro 1H pyrrole
W is H.

In other such compounds,
XisH
Y is Carbonyl group bonded to 5 carbamoyl, 4,5 dihydro 1H pyrrole
Z is carboxyl group bonded to pyridine
W is H.

In other such compounds,
XisH
Y is 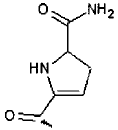
Z is 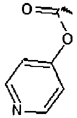
W is H

In other such compounds,
XisH
Y is Carbonyl group bonded to 4,5 dihydro 1H pyrrole 2 carbamoyl
Z is ethylene group
W is Carbonyl group bonded to sec propyl group

In other such compounds,
XisH
Y is 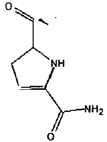
Z is 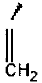
W is

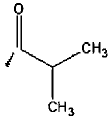

In other such compounds,
XisH
Y is Carbonyl group bonded to 4,5 dihydro 1H pyrrole 2 carbamoyl
Z is Carbonyl group bonded to tertiary butyl group;
W is H.

In other such compounds,
XisH
Y is 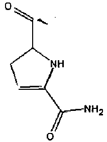
Z is 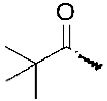
W is H

Also disclosed herein is a pharmaceutical composition, comprising one of the compounds described above or a stereoisomer or tautomer thereof and a pharmaceutically acceptable carrier thereof.

Also disclosed herein is an article of manufacture, comprising: (i) an effective amount of any of compound described above or a stereoisomer or tautomer thereof, or a pharmaceutical composition described above; and (ii) instructions for use in treating a disease, disorder, or condition mediated by KRAS.

In the article of manufacture, the KRAS may comprise a G12C mutant, a G12D mutant, a G13D mutant, or a G12V mutant, or any combination thereof.

The disease, disorder, or condition may be cancer.

The cancer may be non-small-cell lung cancer, colorectal cancer, triple-negative breast cancer, or pancreatic cancer, or any combination thereof.

Also disclosed herein is a kit, comprising: (i) an effective amount of any of compound described above, or a stereoisomer or tautomer thereof, or a pharmaceutical composition described above; and (ii) instructions for use in treating a disease, disorder, or condition mediated by KRAS.

In the kit, the KRAS may comprise a G12C mutant, a G12D mutant, a G13D mutant, or a G12V mutant, or any combination thereof.

The disease, disorder, or condition may be cancer.

The cancer may be non-small-cell lung cancer, colorectal cancer, triple-negative breast cancer, or pancreatic cancer, or any combination thereof.

Also referred herein is a method of modulating, inhibiting, or degrading KRAS in a cell, or any combination of the foregoing, comprising exposing the cell to (i) an effective amount of a compound of compound described above or a stereoisomer or tautomer thereof, or a pharmaceutical composition described above.

The KRAS may comprise a G12C mutant, a G12D mutant, a G13D mutant, or a G12V mutant, or any combination thereof.

Also referred herein is a method of treating a disease, disorder, or condition mediated by KRAS in a subject in need thereof, comprising administering to the subject: (i) an effective amount of compound described above or a stereoisomer or tautomer thereof, or a pharmaceutical composition described above.

The KRAS may comprise a G12C mutant, a G12D mutant, a G13D mutant, or a G12V mutant, or any combination thereof.

The disease, disorder, or condition may be cancer.

The cancer may be non-small-cell lung cancer, triple-negative breast cancer, colorectal cancer, or pancreatic cancer, or any combination thereof.

The chemical structures for the compounds disclosed herein (including the compounds of Formula 1) can be prepared by routine chemistry based on the example structures provided herein.

In one embodiment, an exemplary compound II can be prepared by the Scheme I provided below

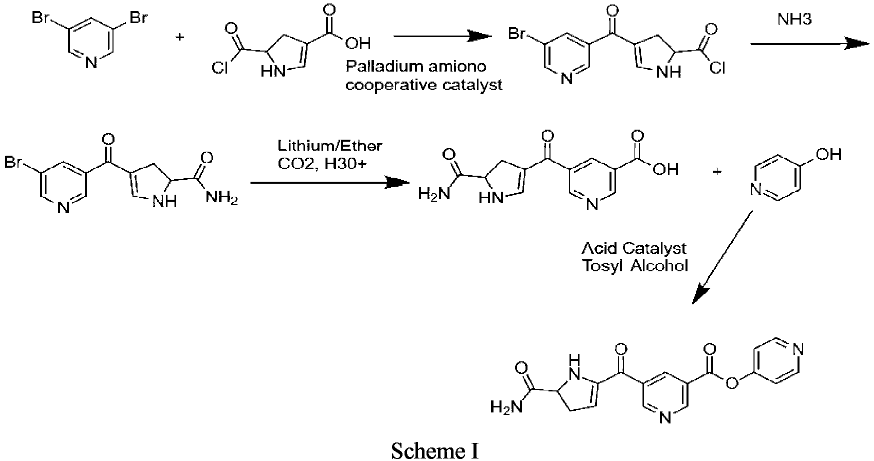

The synthesized compounds of Formula 1 were next subject to molecular characterization to ascertain the structure of the compounds.

### Molecules Characterization:

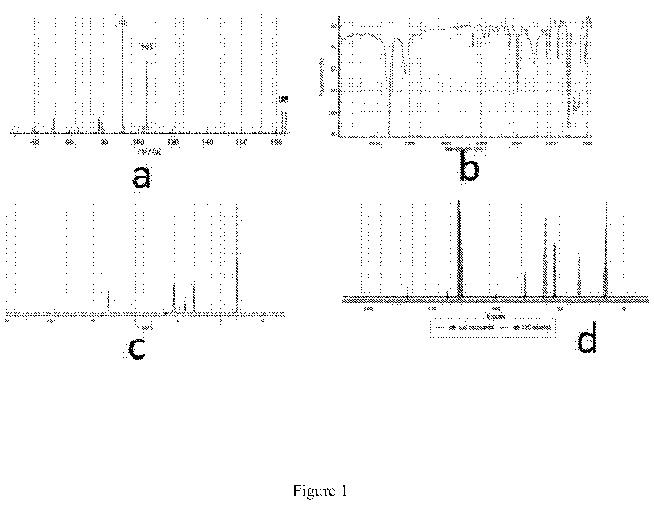

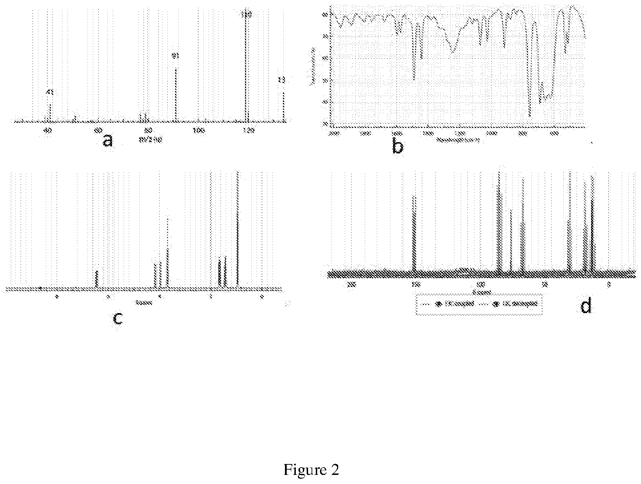

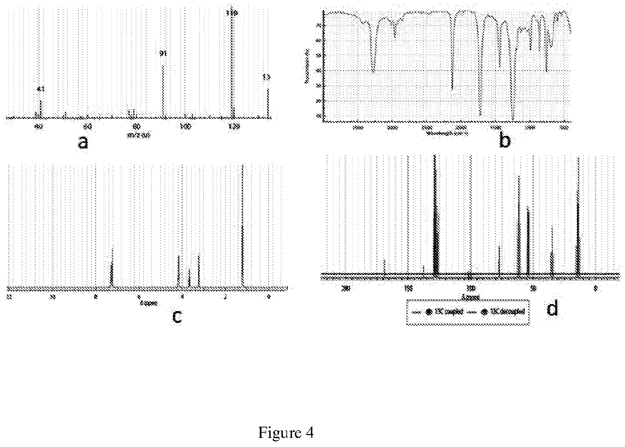

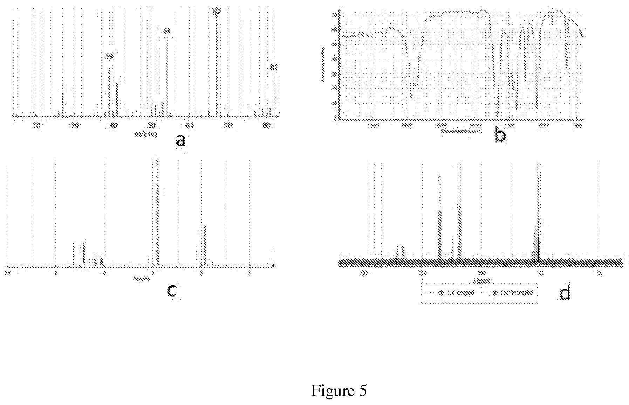

The purity of synthesized products verified by the melting point, Thin Layer Chromatography, HPLC, IR, ¹³C, Mass Spectroscopy and NMR analysis. From Figure 1-5, the structure of the synthesized compounds of Formula 1 is elucidated as Compound I to V as follows in Table 1.

**Table 1:**

| **Compound** | **Structure** |
|---|---|
| 1 | 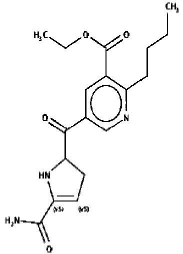 |
| 2 | 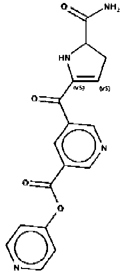 |
| 3 | 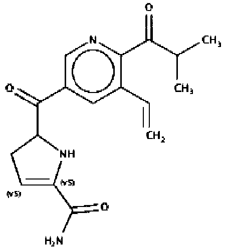 |
| 4 | 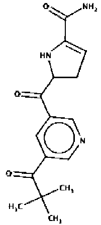 |
| 5 | 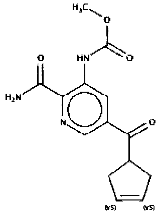 |

In some embodiments, the compounds of Formula 1 and the compounds I- V may encompass both the *cis-* and trans- isomers. In some embodiments, the compounds of Formula 1 and the compounds I- V may be a mixture of *cis-* and trans- isomers. In some embodiments, the compounds of Formula 1 and the compounds I- V may be *cis-* isomer. In some embodiments, the compounds of Formula 1 and the compounds V may be trans- isomer.

In some embodiments, the compound of Formula 1 and the compounds I- V may encompass either R or S stereoisomers and a mixture of stereoisomers. In some embodiments, the compound of Formula (I) may encompass both racemic isomers and enantiomeric isomers
The compounds of the present inventions can be used to perform or provide any of the biological functions, described herein.

### Pharmaceutical Compositions

The present disclosure also includes pharmaceutical compositions comprising a therapeutically effective amount of one or more compounds disclosed herein. In some embodiments, pharmaceutical compositions comprise a therapeutically effective amount of one or more compounds of Formula 1, or pharmaceutically acceptable salts thereof. In other embodiments, pharmaceutical compositions comprise a therapeutically effective amount of one or more compounds selected from Table 1, or pharmaceutically acceptable salts thereof. In some embodiments, the foregoing pharmaceutical compositions further comprise one or more pharmaceutically acceptable excipients.

In various aspects, the amount of compounds of Formula 1 (including compounds in Table 1), or a pharmaceutically acceptable salt thereof, can be administered at about 0.001 mg/kg to about 100 mg/kg body weight (e.g., about 0.01 mg/kg to about 10 mg/kg or about 0.1 mg/kg to about 5 mg/kg). In some embodiments, the compounds herein are administered at between about 200 mg/kg and about 2000 mg/kg-for example, at about 200 mg/kg, at about 500 mg/kg, at about 1000 mg/kg, or at about 2000 mg/kg. In some embodiments, the compounds herein are administered at between about 100 mg/kg and about 600 mg/kg-for example, at about 100 mg/kg, at about 200 mg/kg, at about 300 mg/kg, at about 450 mg/kg, or at about 600 mg/kg.

The concentration of a disclosed compound in a pharmaceutically acceptable mixture will vary depending on several factors, including the dosage of the compound to be administered, the pharmacokinetic characteristics of the compound(s) employed, and the route of administration. The agent may be administered in a single dose or in repeat doses. The dosage regimen utilizing the compounds of the present invention is selected in accordance with a variety of factors including type, species, age, weight, sex and medical condition of the patient; the severity of the condition to be treated; the route of administration; the renal and hepatic function of the patient; and the particular compound or salt thereof employed. Treatments may be once administered daily or more frequently depending upon a number of factors, including the overall health of a patient, and the formulation and route of administration of the selected compound(s).

The compounds or pharmaceutical compositions of the present disclosure may be manufactured and/or administered in single or multiple unit dose forms.

The compounds or pharmaceutical compositions of the present disclosure may be manufactured and/or administered in single or multiple unit dose forms.

The compounds or pharmaceutical compositions of the present disclosure may be administered by various methods including, for example, oral, rectal, buccal, intranasal, and transdermal routes. In certain embodiments, the pharmaceutical composition may be administered intravenously, intraperitoneally, parenterally, intramuscularly, subcutaneously, orally, topically, or as an inhalant.

Compounds and pharmaceutical compositions of the present disclosure may be administered to individuals in any form of generally accepted oral compositions (for example, tablets, coated tablets, gel capsules in a hard or in soft shell, emulsions or suspensions).

Any reference to methods for treatment of the human or animal body by surgery or therapy and diagnostic methods practised on the human or animal body are not part of the invention . Such references serve solely to support the descriptive disclosure of the medical use of the compounds.

### Method of Treatment:

Also disclosed herein are methods of treating a chronic disorder in a human in need thereof, comprising administering the human compounds of formula 1,(I), (II), (III), (IV), or (V), including, for example, Compounds 1-5, or a pharmaceutically acceptable salt thereof. For example, the compounds of the present disclosure (compounds of Formula 1, and Table 1) may be administered to a patient with a chronic condition.

Also disclosed herein is a method of modulating, inhibiting, or degrading KRAS in a cell, or any combination thereof, comprising exposing the cell to (i) an effective amount of a compound of formula 1, (I), (II), (III), (IV), or (V), including, for example, Compounds 1-5, or a stereoisomer or tautomer thereof, or a pharmaceutically acceptable salt of any of the foregoing, or (ii) a pharmaceutical composition, comprising an effective amount of a compound of formula 1, (I), (II), (III), (IV), or (V), including, for example, Compounds 1-5, or a stereoisomer or tautomer thereof, or a pharmaceutically acceptable salt of any of the foregoing, and one or more pharmaceutically acceptable excipients. The method may be a method of modulating KRAS in a cell. The method may be a method of inhibiting KRAS in a cell. The method may be a method of degrading KRAS in a cell. The method may be a method of modulating and degrading KRAS in a cell. The method may be a method of inhibiting and degrading KRAS in a cell. The KRAS may comprise a G12C mutant, a G12D mutant, a G13D mutant, or a G12V mutant, or any combination thereof. For example, the KRAS may comprise a G12C mutant.

Also disclosed herein is a method of treating a disease, disorder, or condition mediated by KRAS in a subject in need thereof, comprising administering to the subject (i) an effective amount of a compound of formula 1, (I), (II), (III), (IV), or (V), including, for example, Compounds 1-5, or a stereoisomer or tautomer thereof, or a pharmaceutically acceptable salt of any of the foregoing, or (ii) a pharmaceutical composition, comprising an effective amount of a compound of formula 1, (I), (II), (III), (IV), or (V), including, for example, Compounds 1-5, or a stereoisomer or tautomer thereof, or a pharmaceutically acceptable salt of any of the foregoing, and one or more pharmaceutically acceptable excipients.

The methods of treatment described herein may lead to a change (%) in the sum of diameters of target lesions in the subject. The change may be measured at day 1, day 14, day 21, day 28, or 6 months after treatment, or any combination of the foregoing (up to 6 cycles). The change may be measured at day 21 of treatment (up to 6 cycles).

The methods of treatment described herein may lead to a reduction in the number of circulating tumor cells in the subject. The methods of treatment may lead to a reduction in the number of circulating tumor cells in a subject with breast cancer, pancreatic cancer, or non-small-cell lung cancer up to 6 months after treatment.

The methods of treatment described herein may lead to a change in the levels of one or more markers of apoptosis in the subject. The methods of treatment described herein may lead to a change in the levels of one or more markers of inflammation in the subject. The methods of treatment described herein may lead to a change in the levels of one or more pathway markers in the subject (as determined by immunohistochemistry (IHC)). The methods of treatment described herein may lead to a change in the immune response in the subject. For example, the methods of treatment described herein may lead to a change in the level of interferon gamma (IFN)-producing T-cells, the cytotoxic T lymphocyte (CTL) response, cytokines (IFN, IL-4, IL-10), or activation markers, or any combination thereof. In some variations of the foregoing, the changes may be measured at day 1, day 14, day 21, day 28, or 6 months after treatment, or any combination of the foregoing (up to 6 cycles).

The disease, disorder, or condition may be mediated by a KRAS G12C mutant, a KRAS G12D mutant, a KRAS G13D mutant, or a KRAS G12V mutant, or any combination thereof. The disease, disorder, or condition may be mediated by a KRAS G12C mutant. The disease, disorder, or condition may be cancer.

In the context of some embodiments of the present invention, the term "chronic disorder" or the term "disease, disorder, or condition" refers to but is not limited to acute lymphoblastic, acute lymphoblastic leukemia, acute lymphocytic leukemia, acute myelogenous leukemia, acute myeloid leukemia, adrenocortical carcinoma, AIDS -related lymphoma, anal cancer, appendix cancer, basal-cell carcinoma, bladder cancer, brain cancer, brainstem glioma, breast cancer, bronchial adenomas/carcinoids, Burkitt's lymphoma, carcinoid tumor, cerebellar or cerebral astrocytoma, cervical cancer, cholangiocarcinoma, chondrosarcoma, chronic lymphocytic or chronic lymphocytic leukemia, chronic myelogenous or chronic myeloid leukemia, chronic myeloproliferative disorders, colon cancer, cutaneous T-cell lymphoma, desmoplastic small round cell tumor, endometrial uterine cancer, ependymoma, esophageal cancer, Ewing's sarcoma, extracranial germ cell tumor, extragonadal germ cell tumor, extrahepatic bile duct cancer, gallbladder cancer, gastric (stomach) cancer, gastrointestinal carcinoid tumor, gastrointestinal stromal tumor (GIST), gestational trophoblastic tumor, glioma of the brain stem, hairy cell leukemia, head and neck cancer, heart cancer, hepatocellular (liver) cancer, Hodgkin lymphoma, hypopharyngeal cancer, hypothalamic and visual pathway glioma, intraocular melanoma, Islet cell carcinoma, Kaposi sarcoma, laryngeal cancer, leukaemia, lip and oral cavity cancer, liposarcoma, lymphoma, male breast cancer, malignant mesothelioma, medulloblastoma, melanoma, Merkel cell skin carcinoma, mesothelioma, metastatic squamous neck cancer, mouth cancer, multiple endocrine neoplasia syndrome, multiple myeloma, multiple myeloma/plasma cell neoplasm, mycosis fungoides, myelodysplastic/myeloproliferative diseases, nasal cavity and paranasal sinus cancer, nasopharyngeal carcinoma, neuroblastoma, non-Hodgkin lymphoma, non-melanoma skin cancer, non- small cell lung cancer, oligodendroglioma, oral cancer, oropharyngeal cancer, osteosarcoma and malignant fibrous histiocytoma, ovarian cancer, ovarian germ cell tumor, ovarian epithelial cancer (surface epithelial- stromal tumor), ovarian low malignant potential tumor, pancreatic cancer, paranasal sinus and nasal cavity cancer, parathyroid cancer, penile cancer, pharyngeal cancer, pheochromocytoma, pineal astrocytoma, pineal germinoma, pineoblastoma and supratentorial primitive neuroectodermal tumors, pituitary adenoma, plasma cell neoplasia, pleuropulmonary blastoma, primary carcinoma, primary central nervous system lymphoma, primary liver cancer, prostate cancer, rectal cancer, renal cell carcinoma, renal pelvis and ureter carcinoma, retinoblastoma, rhabdomyosarcoma, salivary gland cancer, Sezary syndrome, small cell lung cancer, small intestine cancer, soft tissue sarcoma, squamous cell carcinoma, stomach cancer, supratentorial primitive neuroectodermal tumor, testicular cancer, throat cancer, thymoma and thymic carcinoma, thyroid cancer, transitional cell cancer of the renal pelvis and ureter, urethral cancer, uterine sarcoma, vaginal cancer, visual pathway and hypothalamic glioma, vulvar cancer, Waldenstrom macroglobulinemia, Wilms tumor, Parkinson's disease and Parkinsonian disorders, Huntington's disease, Alzheimer's disease, multiple sclerosis, amyotrophic lateral sclerosis, Shy-Drager syndrome, progressive supranuclear palsy, Lewy body disease, spinal ischemia, spinal cord injuries, ischemic stroke, cerebral infarction, spinal cord injury, and cancer-related brain and spinal cord injury, multi-infarct dementia, geriatric dementia, other cognitive impairments, depression, Onychomycosis (fungal infection of the nail, Gingivitis and Periodontal Disease (Gum Disease) obesity and diabetics. In some embodiments, the disease, disorder, or condition is non-small-cell lung cancer, triple-negative breast cancer, or pancreatic cancer, or any combination thereof The compounds of formula 1, (I), (II), (III), (IV), or (V), including any one or more of Compounds 1-5, or a pharmaceutically acceptable salt thereof, may also prove useful in the treatment of SARS and COVID-19 as well as in the treatment of KRAS oncogene mutation in different cancers, such as those described in the list above.

In some embodiments, the chronic condition is cancer. In some embodiments, the cancer is colon cancer, prostate cancer, breast cancer, or leukemia. In some embodiments, the cancer is a stage 4 cancer. In some embodiments, colon cancer, prostate cancer, breast cancer, or leukemia are stage 4. In some embodiment, the chronic condition is KRAS oncogene mutation in different cancers

The compounds and compositions described herein may be administered in combination with one or more of other antibody molecules, chemotherapy, other anti-cancer therapy (e.g., targeted anti-cancer therapies, gene therapy, viral therapy, RNA therapy bone marrow transplantation, nanotherapy, or oncolytic drugs), cytotoxic agents, immune-based therapies (e.g., cytokines or cell-based immune therapies), surgical procedures (e.g., lumpectomy or mastectomy) or radiation procedures, or a combination of any of the foregoing.

Alternatively, or in combination with the aforesaid combinations, the compositions described herein can be administered in combination with one or more of: a vaccine, e.g., a therapeutic cancer vaccine; or other forms of cellular immunotherapy.

The present invention provides a compound or pharmaceutical composition of the present invention for use in a method of treating a disease, disorder, or condition mediated by KRAS, wherein the disease, disorder, or condition is cancer. In some embodiments, the cancer is non-small-cell lung cancer, triple-negative breast cancer, colorectal cancer, pancreatic cancer, or a combination thereof.

The present invention is aimed to invent powerful, therapeutic small molecules that could target mutant KRAS on its active sites. Here we identified a small molecule, compound I, that binds the GTP/GDP-binding pocket of KRAS. For this purpose we studied a strong correlation of measurement among a larger panel of tumor cell lines expressing KRAS mutation and analyses the potency to inhibit the tumor cell growth activated RAS. The growth inhibitory effects of compound I were sustained and irreversible as demonstrated by colony formation and apoptosis assays. Compound I had a good binding affinity to KRAS in vitro and exhibited selective cytotoxicity in oncogenic KRAS expressing cell lines and no or minimum toxicity effect on normal cell lines. Further mechanism study showed that compound I can block the formation of the complex of guanosine triphosphate (GTP) and KRAS in vitro. In addition, compound I inhibited KRAS downstream signaling pathway RAF/MEK/ERK and RAF/PI3K/AKT. compound I induced mitotic arrest as measured by cell cycle analysis of DNA content and phospho-histone H3B immunofluorescence. Further analysis revealed that compound I interfered with localization of the mitosis-inducing protein, PLK1 to kinetochores and decreased nuclear localization of its substrate, Cdc25C, a downstream target of RAS-RAF signaling involved in both mitotic entry and exit checkpoints. compound I also suppresses PD-L1 expression and activates anti-tumor immunity, which may contribute to its antitumor activity and suggests potential benefits of combining with immunotherapy. From these studies, we have identified a novel class of RAS inhibitors that potently and selectively inhibits RAS-driven tumor growth by disrupting downstream signaling, leading to cell cycle arrest and apoptosis. Therefore, compound I may be considered as a potential KRAS inhibitor for treatment of cancer cells carrying KRAS oncogene.

### Articles of Manufacture

Articles of manufacture are also provided herein, wherein the article of manufacture comprises a compound of formula 1, (I), (II), (III), (IV), or (V), or any variation or embodiment thereof, as described elsewhere herein, including, for example, Compounds 1-5, or a stereoisomer or tautomer thereof, or a pharmaceutically acceptable salt of any of the foregoing, in a suitable container. Also provided herein are articles of manufacture, comprising a pharmaceutical composition comprising a compound of formula 1,(I), (II), (III), (IV), or (V), or any variation or embodiment thereof, as described elsewhere herein, including, for example, Compounds 1-5, or a stereoisomer or tautomer thereof, or a pharmaceutically acceptable salt of any of the foregoing, in a suitable container. The container may be a vial, jar, ampoule, preloaded syringe, or intravenous bag.

The present disclosure further provides kits for carrying out the methods described above. The kits may comprise a compound or pharmaceutically acceptable salt thereof as described herein and suitable packaging. The kits may comprise one or more containers comprising any compound described herein. A kit may include a compound of the disclosure or a pharmaceutically acceptable salt thereof, and a label and/or instructions for use of the compound in the treatment of a disease or disorder described herein. The kits may comprise a unit dosage form of the compound.

Provided herein are articles of manufacturing (such as kits), comprising:(i) an effective amount of a compound of formula 1, (I), (II), (III), (IV), or (V), including, for example, Compounds 1-5, or a stereoisomer or tautomer thereof, or a pharmaceutically acceptable salt of any of the foregoing; and (ii) instructions for use in treating a disease, disorder, or condition mediated by KRAS. Also provided herein are articles of manufacturing (such as kits), comprising: (i) a pharmaceutical composition, comprising an effective amount of a compound of formula 1, (I), (II), (III), (IV), or (V),including, for example, Compounds 1-5, or a stereoisomer or tautomer thereof, or a pharmaceutically acceptable salt of any of the foregoing, and one or more pharmaceutically acceptable excipients; and (ii) instructions for use in treating a disease, disorder, or condition mediated by KRAS.

### Examples

### Biological Example 1

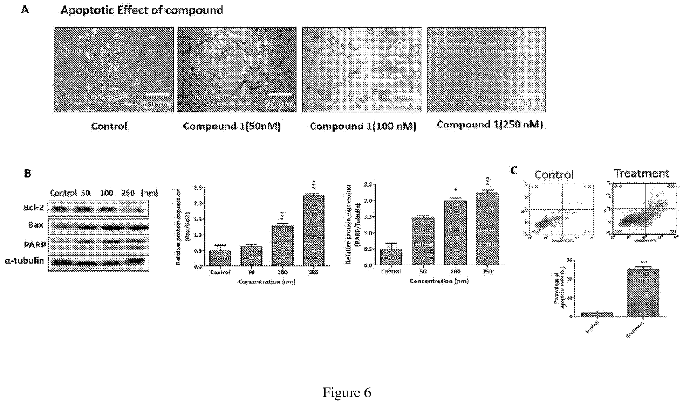

### Compound 1 induces apoptosis in KRAS-dependent cancer cell line (Figure 6)

Compound 1 was subjected to cancer induced model and determined whether the observed growth inhibition was due to apoptosis or necrosis. As illustrated in Fig. 6a, after treatment with Compound 1, Cell morphology of the mutant KRAS cell lines is altered in a concentration-dependent manner, a proportion of cells detached from the culture dish and rounded up, which are predictive apoptotic features. Next, applying standard Annex in V-FITC/PI staining followed by flow cytometry analysis, the percentage of apoptotic and necrotic cells were quantitatively measured. Results showed that Compound 1 significantly induced apoptosis in mutant cancer cells when compared with the untreated cells (Fig. 6C). Furthermore, to examine compound-induced apoptosis in KRAS mutant cells, the expression levels of several well-characterized apoptotic proteins were analyzed by western blotting. Results showed that an increase in the expression of the pro-apoptotic protein Bax and a reduction of expression of anti-apoptotic Bcl-2 were observed in the Compound 1 in-treated cells. In addition, Compound 1 treatment resulted in induction of the cleavage of PARP (Fig. 6B), also consistent with apoptosis. The densitometry quantitation of the ratio of Bax/Bcl2 protein expression and PARP cleavage relative to GAPHD is shown as a bar chart in Fig. 6B. Taken together, these results revealed that Compound 1 induces apoptosis in KRAS mutant cell lines.

### Biological Example 2

### Compound 1 inhibit KRAS mutant expressing cancer cells(Figure 7&12)

It was further examined that if Compound 1 can also induce cytotoxic effects on other cancer cells expressing KRAS mutations, a panel of cancer cell lines which harbor KRAS G12D, G12V and G12C point mutations respectively, were selected and used with KRAS wild-type (WT) controls. As shown in Fig.7 , after treatment with Compound 1 for 24 h, Compound 1 significantly inhibited the cancer cells and stem cells viability in all the mutant cells in a dose-dependent manner and the IC50 values was found to be in nanomolar concentration. Similarly there is minimum toxicity was observed in normal cell lines .In summary, data from the cell lines studies suggested that compound Compound 1 more efficiently inhibits signaling through KRAS consistent with its tight binding to activated KRAS and effect on KRAS-Raf interaction.

The cytotoxicity of Compound 1 was initially evaluated in a panel of 30+ cell lines with wild-type (WT) KRAS or known KRAS mutations. Compound 1 was found to significantly inhibit the cancer cell viability in all the mutant cells in a dose-dependent manner. IC50 values for Compound 1 were found to be in micromolar to nanomolar concentrations (seeFigure 12).

### Biological Example 3

### Structural modeling of Compound 1 in the GTP/GDP-binding pocket of KRAS protein. (Figure 8)

Compound 1 Binds to WT and Oncogenic KRAS Mutants with High Affinity. Figure 8 shows the chemical structure and the predicted complex of compound Compound 1with KRAS, suggesting that the ligand potentially forms multiple favorable interactions with residues in the p1 pocket.

### Biological Example 4

### Compound Compound 1 Blocked GTP-KRAS Formation in KRAS Mutant cells (Figure 9&13)

Actually, mutant KRAS would interfere the balance between GEFs and GAPs, resulting in locking in the active GTP-bound KRAS state and aberrant stimulation of its downstream signaling. Hence, KRAS inhibitors should reduce the formation of GTP-KRAS to disrupt the mutant KRAS function. In order to know whether compound Compound 1 could inhibit activation of KRAS, RAS activation assay was performed to examine the formation of GTP-bound KRAS after treatment with a range of concentrations of compound Compound 1 in k-RAS Mutant cell cells at 24 h, the formation of GTP-KRAS was inhibited in KRAS mutant cells by compound Compound 1 treatment, compared to total amount of KRAS, suggesting this small molecule could partially rescue this unbalance resulted from mutant KRAS.

Compound 1 was found to inhibit the formation of GTP-KRAS (relative to the total amount of KRAS) in KRAS G12C, G12V, G12D, and G13D mutant cells. Compound 1 was found to selectively target mutant forms of KRAS, and no effect was observed in cells with wild-type (WT) KRAS (*see* Figure 13).

### Biological Example 5

### Compound 1 inhibited the Activation of KRAS Downstream Signaling Pathway (Figure 9&14)

Active KRAS stimulates downstream signaling pathways, especially for RAF/MEK/ERK and RAF/PI3K/AKT pathway, and then induces cell proliferation. Therefore, to investigate the effect of Compound 1, the phosphorylation levels of CRAF, AKT and ERK in cell lines was examined to monitor the impact of KRAS signaling by treatment with this compound for 48h.

As expected, the molecules reduced the levels of phosphorylation of CRAF and AKT in a time -dependent manner in KRAS mutant cell lines (Figure 9&14), which indicated that the molecules may block oncogenic KRAS function through inhibiting its downstream signaling pathways.

### Biological Example 6

### Compound 1 suppresses tumor growth in xenograft Model (Figure 10)

The in vivo efficacy of Compound 1 in the context of mutant KRAS was next assessed. Cells were injected into nude mice, and the tumors were allowed to grow to about 60mm³ in size and treated daily with Compound 1 for 14-21 days. Figure: 10 shows that Compound 1 suppressed tumor growth starting at day 9 and showed significant suppression from day 10 to day 14 when compared to the vehicle treated controls. At the day of harvest, the net tumor mass was determined and the average tumor weight of the Compound 1 treatment group was 89% less than the average tumor weight of the control group. The mice (n = 6) exhibited no significant body weight loss or apparent toxicity after treatment with Compound 1. The effect of Compound 1 treatment were further examined on KRAS-mediated RAF, MEK/ERK, and PI3K/AKT cascades in protein extracts derived from vehicle- and Compound 1 -treated tumors. Consistent with the invitro data, it was observed a significant suppression of CRAF, ERK, and AKT phosphorylation. Immunohistochemical analysis also showed that treatment with Compound 1 decreased levels of ERK and AKT phosphorylation, indicating that the growth inhibition induced by Compound 1 is associated with suppression of KRAS mediated signaling. Moreover, immunohistochemistry analysis of the tumors from Compound 1 - treated mice showed a large reduction in cell proliferation as indicated by Ki-67 staining and a prominent increase of apoptotic cells as indicated by cleaved caspase-3 staining. Taken together, these data demonstrated that Compound 1 is effective in suppressing KRAS-driven lung tumor growth.

### Biological Example 7

### Compound 1 Targeting p53 (Figure 11)

A tumor suppressor gene, TP53 (better known as p53) controls several cellular stresses, including DNA damage, hypoxia and oncogene activation. Functionally, the p53 proteins acts as a transcription factor and regulate the gene expression by binding to specific DNA sequences. Classically, p53 has been implicated in controlling genes involved in apoptosis, senescence, cell cycle arrest and play roles in necrosis, autophagy, metabolism, Reactive Oxygen Species (ROS) accumulation and stem cell maintenance. Mutation and loss of p53 is common in variety of cancers including cancer of lung, head and neck, bladder, breast and prostate.

Compound 1 has shown to induce apoptosis by upregulating the expression of p53, Bax, Bak, PUMA, Noxa, and Bimdownregulating Bcl-2, and Bcl-XL and in androgen- dependent and -independent on cancer cells, however, it had no effect on normal fibroblast, epithelial cells. In addition, Compound 1 induces p53 translocation to mitochondria, and Smac release tocytoplasm, which strongly suggests its cancer chemopreventive properties. By altering the p53 and its downstream proteins (p21, cyclin B1, CDK1, Cdc25C) and some apoptosis-related proteins (Bcl-2, Bax, Bid, Bad, Apaf1, AIF and Cyt c), Compound 1 also downregulated the oxidative stress-induced heat shock proteins (HSPs) and histone deacetylase 6 (HDAC6), which further led to apoptosis. Compound 1 showed inhibitory activity on p53-mutated or -overexpressed *in vitro* and *in vivo* preclinical models

### Biological Example 8

### Compound 1 Targeting EGFR and HER2

The activity of Compound 1 on various mutated EGFRs, HER2, and HER4 were examined. MTT assays, flow cytometry, and Western blotting were used to examine the effects of Compound 1 on with different genetic characteristics and relevant molecular mechanisms. Nude mouse xenograft models with cells were used to evaluate the *in vivo* anti-tumor activity of Compound 1. Results showed that Compound 1 effectively inhibited the enzyme activity of EGFR family members, including drug-sensitive EGFR mutations, and EGFR C797S mutations, and wild-type (WT) HER2. Compound 1 blocked EGFR phosphorylation, thereby down regulating downstream PI3K/AKT and MAPK/ERK signaling pathways and inducing G0/G1 arrest in different cells. Compound 1 inhibited tumor growth in mouse xenograft models. In summary, the present findings suggest that Compound 1 has the potential to become an oral anti neoplastic drug for treatment and is worthy of further development.

Due to compromised homologous recombination (HR ) repair, BRCA 1-and BRCA 2-mutated tumours accumulate DNA damage and genomic rearrangements conducive of tumour progression. To identify drugs that target specifically BRCA 2-deficient cells, it was establish that Compound 1 is specifically toxic to BRCA 1/2-deficient cells, cisplatin-resistant ones, suggesting the potential clinical use of Compound 1 against disease which has become resistant to these drugs.

**Table 2: The IC50 of Compound 1 on various breast cells and the corresponding levels of EGFR and HER2 expression as measured by western blot**

| **Cell lines** | **EGFR** | **HER2** | **IC 50 (µM)** |
|---|---|---|---|
| MDA-MB-468 | ++++ | - | 4.21±0.30 |
| BT-549 | ++ | - | 3.24±0.08 |
| MDA-MB-231 | + | - | 4.89±0.37 |
| HCC1937 | + | - | 7.52±1.26 |
| T-47D | + | + | 5.19±0.28 |
| BT-474 | - | ++++ | 4.45±0.10 |
| MDA-MB-453 | - | ++ | 3.65 ±0.44 |
| ZR-75-1 | - | + | 1.69 ±0.14 |
| MCF-7 | - | - | 2.98±0.60 |
| MCF-10 A | +++ | + | 105.56±3.14 |

| | | | |
|---|---|---|---|
| ++++ means very strong expression of EGFR or HER2; +++ means strong expression of EGFR or HER2; ++ means moderate expression of EGFR or HER2; + means low expression of EGFR or HER2; - means no expression of EGFR or HER2 | | | |

### Biological Example 9

### Interaction of Compound 1 with KRAS GTP

Based on the findings, the novel small molecule Compound 1 which competitively bind with GTP binding pockets of K-Ras with high affinity, in switch I region and inhibited the binding of KRAS with its downstream effector such as RAF/PI3K which involved in cancer cells progression. Based on their ability to compete with GTP binding to KRas, block K-Ras signaling, and inhibit cell proliferation in several KRAS tumor-derived human cell lines and also shows 95% reduction in tumor growth inhibition in vivo animal models within 28 days with no evidence of relapse for 6-8 months of times compared with standard drugs.

**Table 3: Interaction of Compound 1 with KRAS GTP**

| Mode | Affinity (kcal/mol) | Dist from rmsd 1.b: | Best mode rmsdu.b |
|---|---|---|---|
| 1 | -7.0 | 0.000 | 0.000 |
| 2 | -6.2 | 14.114 | 174.406 |
| 3 | -6.1 | 3.631 | 6.019 |
| 4 | -6.0 | 4.361 | 8.614 |
| 5 | -5.8 | 12.687 | 17.397 |
| 6 | -5.8 | 13.8963 | 17.013 |
| 7 | -5.8 | 1.346 | 2.159 |
| 8 | -5.6 | 17.986 | 2.667 |
| 9 | -5.6 | 14.986 | 19.948 |

### Biological Example 10

### Effect of Compound 1 downstream signaling-Wild Type

In wild type KRAS, Compound 1 has no effect on GTP binding affinity and the molecules has not altered the expression of the KRAS downstream signalling proteins such as MEK, RAF and PI3K, the effector proteins are intact with highly regulated level of GEF and GAP , which has been observed by protein and Gene Expression analysis. Significant increased expression of Immune cells has been observed in Compound 1 treatment.

**Table 4: Effect of Compound 1 - Downstream Effect-Wild Type**

| Protein | Control | Compound 1 |
|---|---|---|
| pCRAF | 1.52±0.025 | 1.42±0.25 |
| pERK | 1.42±0.069 | 1.46±0.23 |
| pAKt | 1.89±0.63 | 1.72±0.14 |
| pMEK | 1.96±0.22 | 1.85±0.32 |
| pP13K | 1.85±0.32 | 1.62±0.63 |

### Biological Example 11

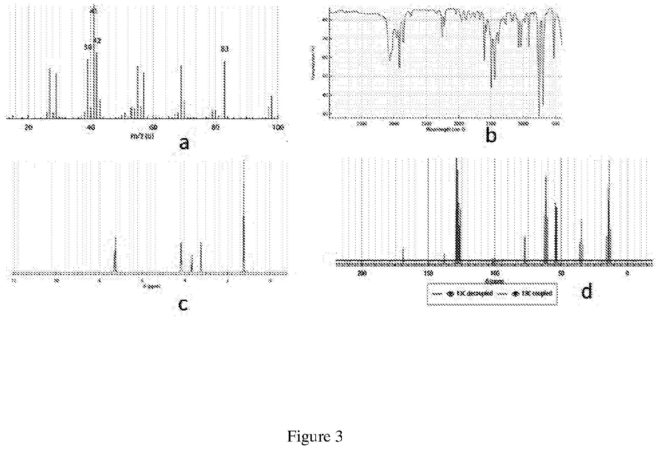

### Fig 3: Effect of Compound 1 on RAS Degradation

Interestingly Compound 1 degrades the RAS via GSK3β phosphorylation through cross talk mechanism with Wnt/β-catenin pathway, via its negative regulators such as APC, axin and glycogen synthase kinase 3β (GSK3β). (Figure 15 &Table 5)

**Table 5: Effect of Compound 1 on RAS degradation**

| Protein | Control | Compound 1 |
|---|---|---|
| GSk3β | 0.69±0.02 | 1.89±0.23 |
| APC | 0.63±0.03 | 0.96±0.44 |
| Axin | 0.25±0.05 | 1.96±0.02 |
| Beta Catenin | 1.32±0.09 | 0.66±0.09 |
| RAS | 1.96±0.06 | 0.25±0.03 |

### Biological Example 12

### Role of miR-30c and miR-21 in RAS Degradation and cancer stem cells.

Mutant KRAS induced a significant upregulation of miR-30c and miR-21. miR-30c and miR-21 are significantly upregulated by both KRAS isoforms and induce drug resistance and enhance cell migration/invasion via inhibiting crucial tumor suppressor genes, such as NF1, RASA1, BID, and RASSF8. The effect of Compound 1 on the expression of miR 30c and miR-21in mutant form of KRAS and Wild type was observed. The microarray analysis showed that the expression of these miR has been significantly reduced in treatment of Compound 1 and significantly increased the expression of tumor suppressor gene in a dose dependent level in mutant form and no significant expression has been observed in wild type. Based on these observation our molecules targets miRNA in KRAS mutant model which acts as an attractive therapeutic tools in cancer medicine, because these miRNA that can silence multiple gene(s) and therefore switch off different pathways simultaneously, which is not possible with protein-based drugs and other covalent inhibitor of different KRAS mutant form, and eventually Compound 1 has no toxicity or off target effects compared to other standard drugs

**Table 6 : Effect of Compound 1 in miRNA upregulation**

| Protein | Control | Compound 1 |
|---|---|---|
| miR30c | 0.96±0.025 | 1.89±0.25 |
| miR21 | 0.72±0.02 | 1.58±0.32 |
| NF1 | 1.56±0.06 | 1.21±0.033 |
| RASA1 | 1.69±0.36 | 0.32±0.031 |
| BID | 1.56±0.36 | 0.32±0.012 |

### Biological Example 13

### Oral Bioavailability Data/Crossing Blood-Brain Barrier Data for Compound 1

**Table 7: Pharmacokinetic Data for Compound 1**

| | | |
|---|---|---|
| | | Cmax=2.6 µg/mL (10mg/kg b.wt. mice) |
| | | Cmax=1.9 µg/mL (10mg/kg b.wt rat) |
| | T max =2h (mice 10 mg/kg,b.wt;) | |
| Pharmacokinetics | T max =2h (rats, 10 mg/kg,b.wt; ) | |
| | T_{1/2} = 5.8h (mice) | |
| | T_{1/2} = 9.8h (rats) | |
| | F-92% (Bioavailability) | |

**Table 8: Crossing Blood-Brain Barrier Data for Compound 1**

| Biosamples | *T*1/2 (h) | *C*max (ng/g, ng/mL) | *T*max (h) | *AUC*0-*t*_D (h×ng/g/mg, h×ng/mL/mg) | *MRT*0-*t* (h) |
|---|---|---|---|---|---|
| Brainstem | 1.81±0.23 | 185.74±3.46 | 1.35±0.33 | 85.63±17.43 | 5.89±0.34 |
| Hypothalamus | 1.18±0.26 | 203.88±42.33 | 0.54±0.24 | 59.10±5.06 | 5.25±0.60 |
| Hippocampus | 1.19±0.22 | 223.48±34.72 | 1.25±1.08 | 58.20±15.36 | 5.18±0.51 |
| Cortex | 5.86±2.91 | 276.52±48.17 | 1.14±0.09 | 152.08±5.25 | 6.23±0.65 |
| Striatum | 3.45±0.93 | 200.02±30.90 | 0.14±0.09 | 123.81±4.90 | 6.75±0.68 |

### Biological Example 14

### Comparison of Compound 1 with known KRAS inhibitors

The reactivation of RAS signaling was compared for the administration of Compound 1 and two known KRAS inhibitors (AMG510 and MRTX849). In all KRAS G12C mutant cell lines, Compound 1, AMG510, and MRTX849 suppressed MAPK pathway signaling, a key downstream effector pathway of KRAS, as measured by inhibition of phosphor-MEK, phosphor-ERK, and phosphor-AKT at 4 hours. By 24-48 hours, the RAS-MAPK pathway signaling began to rebound in cells treated with AMG510 and MRTX849, leading to pathway reactivation and incomplete suppression of pMEK, pERK, pAKT, and MYC by 72 hours. The rapid and consistent reactivation of signaling observed following KRAS G12C inhibition suggests that adaptive feedback may have the potential to limit the efficacy of these inhibitors. In contrast, administration of Compound 1 resulted in a significant reduction in the phosphorylated forms and no rebound activity was observed by 72 hours (*see* Figure 16). Densitometry of phosphor-ERK, MEK, AKT, and MYC is normalized to GAPDH. Results represent an average across 8 cell lines.

The cytotoxic effect of Compound 1, AMG510, and MRTX849 on an NCI-Panel of 10 KRAS G12C mutant cell lines was also compared. Compound 1 induced apoptosis in a dose-dependent manner and showed a significant reduction in the number of cancer cells when compared against AMG510 and MRTX849 (*see* Figure 17).

### Biological Example 15

### Compound 1: In vivo tumor model

Compound 1 was found to cause regression of all mutant-KRAS-driven cancer in immune-competent mice. Compound-1-supressed tumor growth started at day 9 and showed significant suppression from day 10-14 in all the mutant-KRAS-expressing animal models. Compound 1 was also found to inhibit downstream signaling. Treatment with Compound 1 showed a 95% reduction in tumor growth within 28 days and no evidence of relapse for 6-8 months. See Figure 18 and Figure 19.

### Biological Example 16

### Compound 1: Toxicity Studies

Histopathology of harvested normal tissues (brain, heart, lung, liver, spleen, kidney, and intestine) revealed no evidence of normal tissue toxicities after treatment with specific doses of Compound 1. Tests of blood cells (WBC, RBC, and PLT) for bone marrow, RFT for kidney, and ALT/AST for liver functions all provided results in the normal range. *See* Figure 20.

### Biological Example 17

### Compound 1: Clinical Trial Protocol

This study is an open label, two-part, First in Human (FIH) dose-finding study designed to determine the safety, tolerability, Pharmacokinetics (PK), Pharmacodynamics (PD) and proof-of-concept (POC) of Compound 1 in patients with advanced or metastatic solid tumors. The study consists of two parts:
Part 1: Dose escalation in patients with advanced or metastatic solid tumors, including Compound 1 dose levels. This study plans to start dose escalating from 50 mg, followed by 100 mg, 200 mg, 300 mg, 450 mg, and 600 mg as tentatively designated escalating dose groups. Approximately 11 to 24 patients in total are enrolled in Part 1, covering 5 dose levels.

The Primary Objective is determining the safety and tolerability of Compound 1 and defining an appropriate dosefor further evaluation in Part 2.

The study starts with an accelerated-titration dose-escalation scheme enrolling one evaluable patient per cohort for the first 2 dose levels; pending safety signals followed by a classic 3+3 design.

Part 2: Dose expansion where at least 3 parallel groups of patients with advanced Non-Small-Cell Lung Cancer (NSCLC), Triple Negative Breast Cancer (TNBC) and Pancreatic cancer (PANC) are treated at the recommended Phase 2 dose (RP2D) of Compound 1 to further characterize the safety, tolerability, PK, PD and antitumor activity of Compound 1.

Approximately 18 (15 evaluable) patients are enrolled in each of the 3 parallel groups of patients (NSCLC, TNBC, PANC) in Part 2.

Compound 1 is administered through once daily continuous dosing with dose escalation to maximally-tolerated dose (MTD) until progression or discontinuation. Each cycle of the study lasts 28 days. The study can be extended by up to 6 cycles for confirmation of safety and efficacy.

From the above biological examples it is clearly inferred that the Compound 1 as a new class of KRAS that selectively targets the GTP/GDP binding pocket. The binding of Compound 1 to 5 with KRAS promotes accumulation of GTP-KRAS probably by prevention of cleavage from GTP into GDP. Compound 1 to 5 -induced hyperactivation of mutant KRAS facilitates apoptotic cell death in mutant KRAS cancer cells. Combining the results with detailed structural analysis, we are able to describe key ligand-receptor interactions that correlate with activity. Thus, showing that our screening techniques were very successful at generating KRAS binders that have effects on signaling in cells. To our knowledge compound Compound 1 is the first known nanomolar binder of KRAS that disrupts interaction with CRaf resulting in decreased p-ERK levels and cell proliferation. Therefore, compound Compound 1 is a promising hit for the development of novel non-covalent KRAS inhibitors. The development of this new class of anticancer drug offers a potentially effective strategy for the treatment of cancer with KRAS mutation and/or mutant KRAS-driven cancer. Furthermore Compound 1 inhibits KRAS GTP and activates degradation via GSK3 β through ubiquitin mediated pathway via miR 30c and miR 21 regulation which leads to programmed cell death.

From the foregoing, it will be appreciated that various embodiments of the present disclosure have been described herein for purposes of illustration, and that various modifications may be made without departing from the scope of the present disclosure. Accordingly, the various embodiments disclosed herein are not intended to be limiting, with the true scope being indicated by the following claims.

## Claims

1. A compound of formula 1 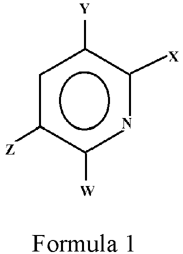
or pharmaceutically acceptable salt thereof, or combination thereof
wherein:
X is C₁-C₅ alkyl;
Y is carboxyl group bonded to C₁-C₅ alkyl;
Z is carbonyl group bonded to 5-carbamoyl-2,3-dihydro-1H-pyrrole; and
W is H.

2. The compound of claim 1, wherein the compound is

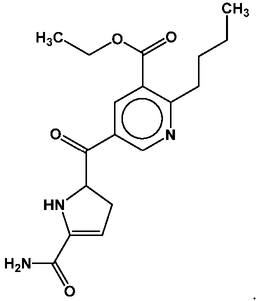

3. A pharmaceutical composition comprising an effective amount of a compound of claim 1 or claim 2 and a pharmaceutically acceptable carrier.

4. The compound of claim 1 or 2 or the pharmaceutical composition of claim 3, for use in a method of treating a disease, disorder, or condition mediated by KRAS, wherein the disease, disorder, or condition is cancer.

5. The compound or pharmaceutical composition for use of claim 4, wherein the cancer is non-small-cell lung cancer, triple-negative breast cancer, colorectal cancer, pancreatic cancer, or a combination thereof.

## Patentansprüche

1. Verbindung der Formel 1 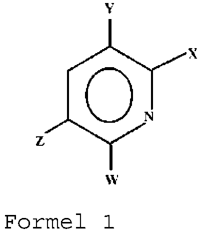
oder pharmazeutisch unbedenkliches Salz davon oder Kombination davon,
wobei:
X für C₁-C₅-Alkyl steht;
Y für eine Carboxylgruppe steht, die an C₁-C₅-Alkyl gebunden ist;
Z für eine Carbonylgruppe steht, die an 5-Carbamoyl-2,3-dihydro-1H-pyrrol gebunden ist; und
W für H steht.

2. Verfahren nach Anspruch 1, wobei es sich bei der Verbindung um Folgendes handelt:

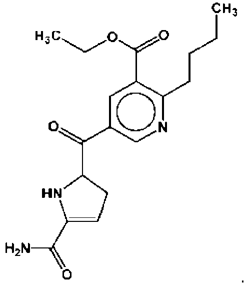

3. Pharmazeutische Zusammensetzung, umfassend eine wirksame Menge einer Verbindung nach Anspruch 1 oder Anspruch 2 und einen pharmazeutisch unbedenklichen Träger.

4. Verbindung nach Anspruch 1 oder 2 oder pharmazeutische Zusammensetzung nach Anspruch 3 zur Verwendung bei einem Verfahren zum Behandeln einer Krankheit, Störung oder eines Leidens, die/das durch KRAS vermittelt wird, wobei es sich bei der Krankheit, Störung oder dem Leiden um Krebs handelt.

5. Verbindung oder pharmazeutische Zusammensetzung zur Verwendung nach Anspruch 4, wobei es sich bei dem Krebs um nicht kleinzelligen Lungenkrebs, dreifach negativen Brustkrebs, Kolorektalkrebs, Bauchspeicheldrüsenkrebs oder eine Kombination davon handelt.

## Revendications

1. Composé de formule 1 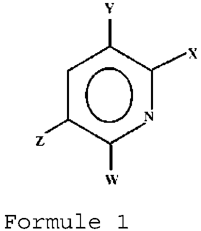
ou sel pharmaceutiquement acceptable de celui-ci, ou combinaison de ceux-ci
dans laquelle :
X est C₁-C₅ alkyle ;
Y est un groupe carboxyle lié à C₁-C₅ alkyle ;
Z est un groupe carbonyle lié à 5-carbamoyl-2,3-dihydro-1H-pyrrole ; et
W est H.

2. Composé selon la revendication 1, dans lequel le composé est

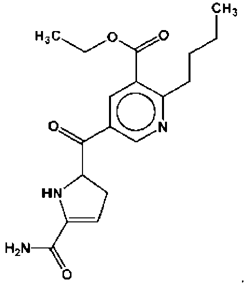

3. Composition pharmaceutique comprenant une quantité efficace d'un composé selon la revendication 1 ou la revendication 2 et un support pharmaceutiquement acceptable.

4. Composé selon la revendication 1 ou 2 ou composition pharmaceutique selon la revendication 3, pour utilisation dans un procédé de traitement d'une maladie, d'un trouble ou d'une affection médiée par KRAS, dans lequel la maladie, le trouble ou **l'affection** est un cancer.

5. Composé ou composition pharmaceutique pour utilisation selon la revendication 4, dans lequel le cancer est un cancer du poumon non à petites cellules, un cancer du sein triple négatif, un cancer colorectal, un cancer du pancréas, ou une combinaison de ceux-ci.
